(19) Europäisches Patentamt / European Patent Office / Office européen des brevets

(11) **EP 2 843 413 B1**

(12) **EUROPEAN PATENT SPECIFICATION**

(45) Date of publication and mention
of the grant of the patent:
**06.05.2020 Bulletin 2020/19**

(51) Int Cl.:
***G01N 33/66*** *(2006.01)*   ***A61B 5/00*** *(2006.01)*
***A61B 5/145*** *(2006.01)*

(21) Application number: **13781052.9**

(22) Date of filing: **23.04.2013**

(86) International application number:
**PCT/JP2013/061859**

(87) International publication number:
**WO 2013/161796 (31.10.2013 Gazette 2013/44)**

(54) **BLOOD GLUCOSE METER**

BLUTZUCKERMESSGERÄT

GLYCOMÈTRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.04.2012 JP 2012098366**

(43) Date of publication of application:
**04.03.2015 Bulletin 2015/10**

(73) Proprietor: **Terumo Kabushiki Kaisha
Tokyo 151-0072 (JP)**

(72) Inventor: **OHASHI, Hirotaka
Tokyo 103-0028 (JP)**

(74) Representative: **Casalonga
Casalonga & Partners
Bayerstraße 71/73
80335 München (DE)**

(56) References cited:
**EP-A1- 2 345 893      WO-A1-2005/106446
WO-A1-2006/072035      WO-A1-2011/041007
JP-A- 2003 159 234      JP-A- 2006 071 421
JP-A- 2010 042 261      JP-A- 2011 064 596
JP-A- 2011 064 597      JP-A- 2012 018 010
US-A1- 2009 240 127**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to a blood glucose meter for measuring a blood glucose level of a patient and displaying the blood glucose level on a display screen.

Background Art

It is known that diabetes causes abnormal secretion of insulin and hyposensitivity to insulin in a pancreas. In particular a patient of type 1 diabetes who lost insulin secretion needs to measure a blood glucose level before meals to determine an amount of insulin to be administered.

The applicant has developed a small-sized blood glucose level measuring device (hereinafter referred to as "blood glucose meter") allowing a patient or a family member to measure a blood glucose level at home, which is being manufactured and sold.

Patent application of the blood glucose meter by the applicant is listed in Patent Literature 1.

Relevant documents to understand the background of the invention are also US2009/240127 (A1), EP2345893 (A1), WO2006/072035 (A1).

Citation List

Patent Literature

Patent Literature 1: JP 2011-64596 A

Summary of Invention

As widely known, diabetes is at present a disease that is very difficult to recover from unless innovative progress is made in medical technology such as regenerative medicine. Diabetics have to manage to keep their blood glucose levels always in an appropriate range on a daily basis. Moreover, diabetics have to periodically see doctors to report the measured result of blood glucose levels to get advice on their lifestyle. When a doctor sees a patient, the doctor checks the data of blood glucose level of the patient, which is measured with the patient's blood glucose meter, accumulated for a period of time of a few weeks to a few months. The blood glucose meter therefore has a history display function which allows a user to check accumulated data of blood glucose level on a built-in liquid crystal display.

A conventional blood glucose meter only has a function to display data of the past blood glucose level of a patient on a liquid crystal display. However, since diabetes is a chronic disease, it is important for a patient to know the change in the condition of a disease during a certain period of time whether the condition of the disease is getting better or getting worse so that the patient can understand the importance of the change in lifestyle. Conventional blood glucose meters cannot provide such information telling medium and long-term change in the condition of a disease.

The present invention is made in view of such issue. The object of the present invention is to provide a blood glucose meter having a function to display medium and long-term change in a blood glucose level on a display screen at a suitable timing.

To solve the problem described above, the blood glucose meter according to the present invention includes a blood glucose level measuring unit for measuring a blood glucose level of a patient, a blood glucose level table including a blood glucose level field storing the blood glucose level of the patient measured with the blood glucose level measuring unit and a date and time field storing a date and time when the blood glucose level measuring unit measured the blood glucose level of the patient, a display unit for displaying the blood glucose level stored in the blood glucose level field, and a control unit giving a command to supply power to the display unit. The blood glucose meter according to the present invention further includes an input/output controller, an input/output controller configured to perform, in sequence, power supply to the display unit according to a command given through the control unit, reading the blood glucose level measured during a predetermined period of time from the blood glucose level table to calculate an average value, comparing the average value with a first threshold and a second threshold, and displaying image information on the display unit according to the compared result.

The blood glucose meter according to the present invention displays on the display screen the evaluation result based on an average value of blood glucose level measured during a certain period of time until the present at startup and just before shutdown. This allows the user to understand the change in blood glucose level at a glance.

Brief Description of Drawings

Figs. 1A to 1C are external views of a blood glucose meter as an exemplary construction of the present invention. Fig. 1A is a perspective view, Fig. 1B is a top view, and Fig. 1C illustrates the blood glucose meter with a cover detached.
Fig. 2 is a block diagram illustrating a configuration of the blood glucose meter.
Fig. 3 is a schematic chart showing an example of a field structure and a record of a blood glucose level table.
Fig. 4 is a flowchart illustrating a flow of a sleep process of the blood glucose meter.
Fig. 5 is a flowchart illustrating a flow of a main process of the blood glucose meter.
Fig. 6 is a flowchart illustrating a flow of a blood glucose level evaluation displaying process.
Fig. 7 illustrates an example of a setting screen for graph display on a liquid crystal display.
Figs. 8A to 8C illustrate examples of graph display on the liquid crystal display.
Fig. 9 illustrates an example of a graph display on the liquid crystal display.
Figs. 10A to 10E illustrate examples of face illustrations displayed on the liquid crystal display by five-point rating.
Fig. 11 is a schematic chart showing an example of a field structure and a record of a blood glucose level table in a meal-button mode.
Figs. 12A and 12B are block diagrams of the configuration of hardware including the blood glucose meter and a personal computer.

Best Mode for Carrying Out the Invention

A blood glucose meter according to an embodiment will schematically be described.
As well-known, highly developed electronic devices can perform advanced functions with low power consumption. Thus, the inventor intends to develop a blood glucose meter as a tool providing further useful information to improve QOL (quality of life) of diabetics in addition to the conventional functions of measuring only a blood glucose level.
The inventor proposes to provide two pieces of information to improve QOL of diabetics.
One is a five-point rating evaluation result of a medium-term moving average value of blood glucose level.
Another one is a graph showing medium and long-term change in blood glucose level, which is a graph being provided at predetermined timings, namely every week, every one month, and every three months.
However, a patient usually will not use a function to display such information if the patient has to give a command to the device by himself/herself.
Regarding such issue, a blood glucose meter is configured to always display such information just after a startup and just before a shutdown.

External Appearance

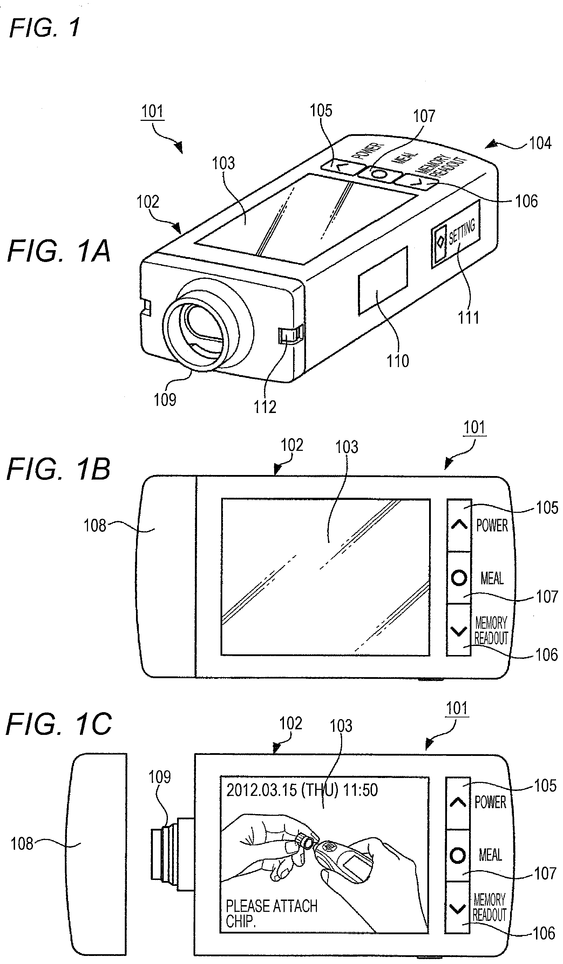

Figs. 1A to 1C are external views of a blood glucose meter 101 as an example of an exemplary construction of the present invention. Fig. 1A is a perspective view, Fig. 1B is a top view, and Fig. 1C illustrates the blood glucose meter with a cover detached.
As illustrated in Fig. 1A, a blood glucose meter 101 includes a liquid crystal display 103 displaying information in color on the face of a housing 102 and control buttons configured with well-known membrane switches. The control buttons 104 are provided on a top face and a side face of the housing 102. That is, as illustrated in Figs. 1B and 1C, an up button 105, a down button 106, and a determination button 107 are provided on the top face of the housing 102, and a set button 111 is provided on the side face of the housing 102. Further, a cap 110 of a serial interface terminal configured with a well-known MicroUSB is provided on the side face of the housing 102.
The control button 104 generally represents the up button 105, the down button 106, the determination button 107, and the set button 111.
The up button 105 for moving upward a cursor displayed on the liquid crystal display 103, the down button 106 for moving the cursor downward, and the determination button 107 for giving commands such as "execute" and "determine" are provided on the face of the housing 102. The set button 111 for adjusting various settings is provided on the side face of the housing 102.
The up button 105 is pushed for about 1.5 seconds or more (hereinafter expressed as "give a long-push") to power on or power off.
By giving a long-push to the down button 106, the blood glucose level stored in a blood glucose level table, which will be described below, is displayed.
By giving a long-push to the determination button 107, an "after-meal flag" or a "meal record", which will be described below, is recorded in the blood glucose level table.

When the blood glucose meter 101 is in an "after-meal flag" mode, the determination button 107 functions as an "after-meal button" to record an after-meal flag in the corresponding record area in the blood glucose level table.

When the blood glucose meter 101 is in a "meal flag" mode, the determination button 107 functions as a "meal button" to record the meal record in the blood glucose level table.

The "after-meal flag" mode and the "meal flag" mode can be switched by following the instruction of a setting menu displayed by pushing the set button 111.

On one of short sides of the housing 102 of the blood glucose meter 101, an optical measurement unit 109 is provided which is protected by a detachable cover 108. A protrusion (not shown in the drawing) is provided in the inner side of the cover 108. By the protrusion engaging with a recess 112 provided in the housing 102, a built-in micro switch (not shown in the drawing) in the housing 102 is switched off.

The blood glucose meter 101 operates by battery such as a well-known lithium ion battery is configured to be set to a sleep mode to save power when not in a state of operation. When the blood glucose meter 101 is in the sleep mode, the blood glucose meter 101 returns to an active mode activating all the functions of the blood glucose meter 101 by giving a long-push to the up button 105 or taking off the cover 108 to switch on the micro switch. Hereinafter, a state in the sleep mode is referred to as "power-off state", and a state in the active mode is referred to as "power-on state".

As illustrated in Fig. 1C, when the state changes to the power-on state by taking off the cover 108, a guidance to attach a tip is displayed on the liquid crystal display 103 so that blood glucose level can immediately be measured. An illustration and an instruction displayed on the liquid crystal display 103 in Fig. 1B give guidance to attach the tip.

Functional Structure

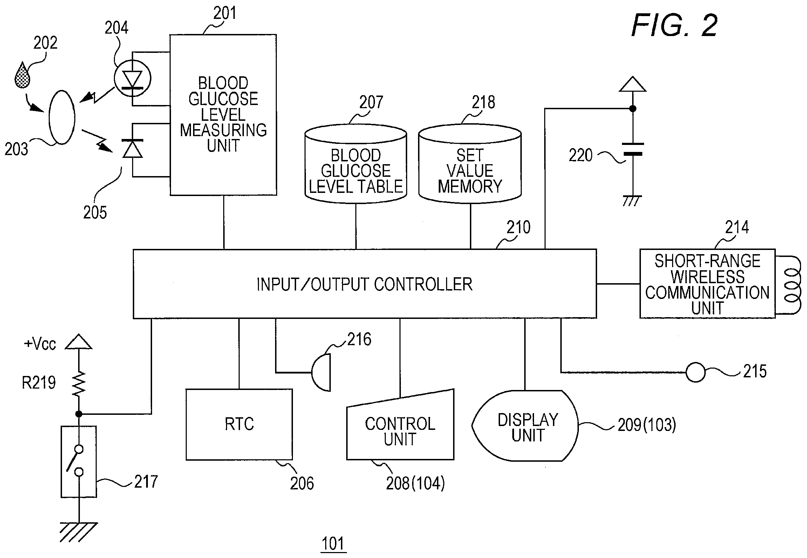

Fig. 2 is a block diagram illustrating a configuration of the blood glucose meter 101.

The blood glucose meter 101 is an electronic device including a well-known microcomputer as a major part. The microcomputer reads a program stored in a ROM (not shown in the drawing) to construct a portion of the structure illustrated in Fig. 2.

A blood glucose level measuring unit 201 detects the change in optical reflectivity of a test paper 203, which changes color by making contact with blood 202 of a person to be measured, to measure a blood glucose level.

The blood glucose meter 101 is configured to measure a blood glucose level in a basically similar manner to the conventional art. The configuration to measure a blood glucose level will briefly be described below.

The blood 202 of the person to be measured is suctioned by a measuring tip (not shown in the drawing) attached to the blood glucose meter 101. The measuring tip includes therein the test paper 203 made of porous membrane such as polyether sulfone. When the blood 202 suctioned by the measuring tip reaches the test paper 203, the blood 202 reacts with a reagent included in the test paper 203 to change color. The test paper 303 is then lightened with light emitted from an LED 204, or a light-emitting element. A photodiode 205 which is a light-receiving element receives the reflected light from the test paper 203. After a predetermined reaction time, an analogue received light intensity signal obtained in the photodiode 205 is converted at an A/D converter (not shown in the drawing) into a digital value which is then converted into a blood glucose level.

The configuration to measure a blood glucose level is not limited to the optical measurement using coloring reagents as described above. Conventional configurations to measure a blood glucose level such as measurement with an electrochemical sensor can be used.

A real time clock (hereinafter referred to as "RTC") 206 implemented in many typical microcomputers outputs date and time information. The RTC 306, supplied with power even under the power-off state, outputs correct date and time information.

A date and time of measurement, a measured blood glucose level, and an after-meal flag, which will be described later, are recorded in a blood glucose level table 207. The blood glucose level table 207 is provided in a nonvolatile storage (not shown in the drawing) such as an EEPROM.

A control unit 208 is provided as the control button 104 in Fig. 1, and a display unit 209 is provided as a liquid crystal display 103 in Fig. 1.

The input/output controller 210 records the blood glucose level data obtained from the blood glucose level measuring unit 201 and the date and time of measurement obtained from the RTC 206 in the blood glucose level table 207. Further, the input/output controller 210 displays the measured blood glucose level on the display unit 209 and performs various kinds of data processing responding to commands given through the control unit 208.

A short-range wireless communication unit 214 is used to transmit the blood glucose level data recorded in the blood glucose level table 207 to a personal computers or the like.

A serial interface 215 is used to transmit the blood glucose level data recorded in the blood glucose level table 207 to an external device such as a personal computer.

When a long-push is given to the meal button, namely the determination button 107, the input/output controller 210 gets the present date and time information from the RTC 306 and after two hours, sets off a buzzer 216.

The input/output controller 210 controls the power supplied to the display unit 209 and the blood glucose level measuring unit 201.

The input/output controller 210 will not supply power to the display unit 209 and the blood glucose level measuring unit 201 from a battery 220 in the power-off state.

When the up button 105 receives a long-push or a micro switch 217 is switched on by removing the cover 108, the input/output controller 210 changes to the power-on state. Then the input/output controller 210 supplies power to the display unit 309 and the blood glucose level measuring unit 201 from the battery 220.

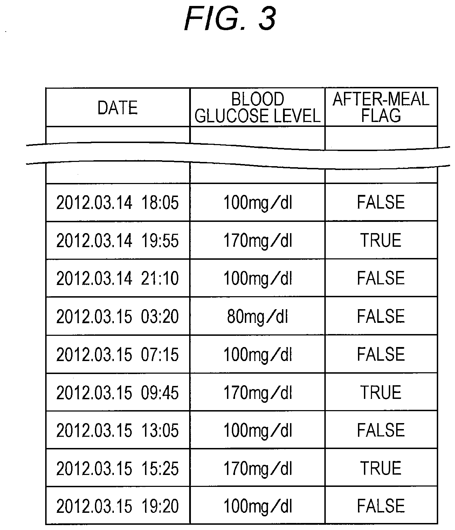

Fig. 3 is a schematic chart showing an example of a field structure and a record of the blood glucose level table 207.

The date and time field stores the date and time information at a point of time when a blood glucose level is measured.

The blood glucose level field stores a measured blood glucose level.

When a blood glucose level is measured, the after-meal flag field stores, by giving a long-push to the determination button 107, an after-meal flag indicating that the blood glucose level is measured after a meal.

Records of a blood glucose level are chronologically recorded in the blood glucose level table 207. Therefore, the latest record is recorded last.

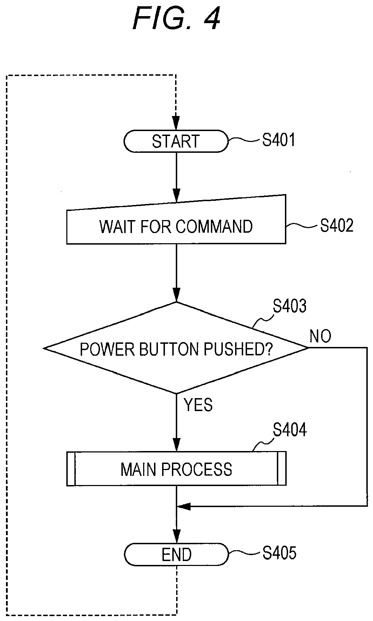

Fig. 4 is a flowchart illustrating a flow of a sleep process of the blood glucose meter 101.

When the processing starts (S401), the input/output controller 210 of the blood glucose meter 101 cuts off power supply to large power consuming devices such as the A/D converter (not shown in the drawing), the display unit 209, and an LED 304, and changes to the sleep mode to operate under a low frequency clock between a few hundreds kHz to a few MHz. The command given to the control unit 208 and the state of the micro switch are detected by the input/output controller 310 (S402).

The input/output controller 210 determines whether a command given to the control button 104 is a long-push given to the up button 105, that is whether a power button is pushed, and whether the micro switch is pushed on (S403).

When the power button is pushed or the micro switch is pushed on (YES in S403), the input/output controller 210 executes a main process (S404), and the process flow ends (S405).

When the power button is not pushed and the micro switch is not pushed on (NO in S403), the process flow ends (S405) without any process executed by the input/output controller 210. The sleep process is a loop operation which repeats from the start after the end of the process.

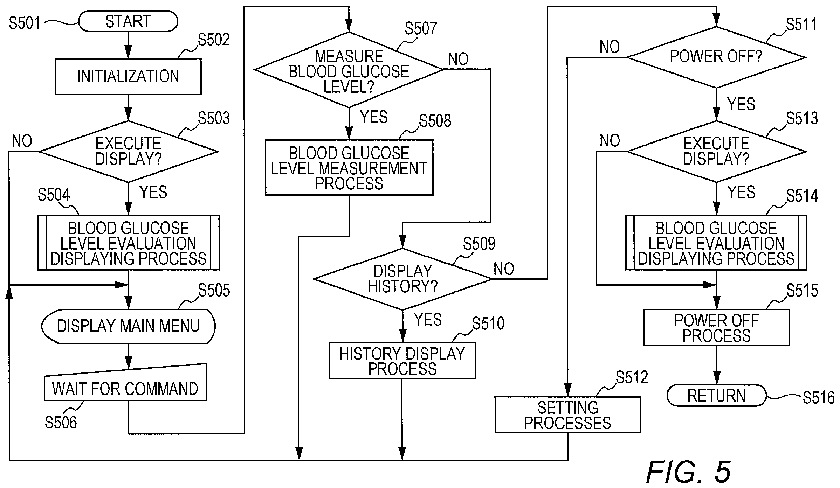

Fig. 5 is a flowchart illustrating in detail a flow of the main process of the blood glucose meter 101 in step S404 in Fig. 4.

When the process starts (S501), the input/output controller 210 executes a predetermined initiation process such as supplying power to large power consuming devices such as the display unit 209 and the LED 204 (S502). The input/output controller 210 then refers setting information stored in a set value memory 218 to determine whether to execute information display at startup (S503).

If the information display is set to be executed at startup (YES in S503), the input/output controller 210 executes a blood glucose level evaluation displaying process (S504). The input/output controller 210 then displays a main menu on the display unit 209 (S505) to wait for a command given through the control unit 308 (S506).

If the information display is not set to be executed at startup in step S603 (NO in S603), the input/output controller 310 will not execute the blood glucose level evaluation displaying process in step S504 and immediately displays the main menu on the display unit 309 (S505) to wait for a command given through the control unit 208 (S506).

If a command is given to the control button 104 to measure a blood glucose level (YES in S507), the input/output controller 210 executes a blood glucose level measurement process (S508) and then displays the main menu again (S505).

If a command is given to the control button 104 to display the history of the past blood glucose level (YES in S509) instead of measuring a blood glucose level (NO in S507), the input/output controller 310 executes a history display process (S510) and then displays the main menu again (S505).

If a command is given to the control button 104 to determine settings (NO in S511) instead of displaying the history of the past blood glucose level (NO in S509), the input/output controller 310 executes setting processes (S612) and then displays the main menu again (S505).

If a command is given to the control button 104 to power off (YES in S513) instead of determining settings, the input/output controller 210 refers setting information stored in the set value memory 218 to determine whether to execute information display at shutdown (S613). If the information display is set to be executed at shutdown (YES in S513), the input/output controller 310 executes a blood glucose level evaluation displaying process (S514). The input/output controller 310 then executes a power off process (S515) to finish the series of process (S516).

If the information display is not set to be executed at shutdown in S513 (NO in S513), the input/output controller 210 will not execute the blood glucose level evaluation displaying process in step S514 and immediately executes a power off process (S515) to finish the series of process (S516).

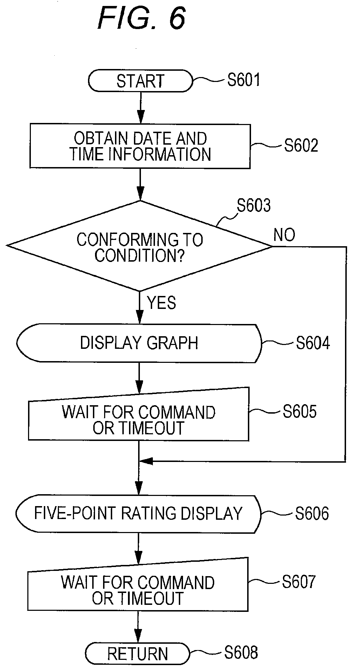

Fig. 6 is a flowchart illustrating a detailed flow of the blood glucose level evaluation displaying process in steps S504 and S514 in Fig. 5.

When the process starts (S601), the input/output controller 210 first obtains present date and time information from the RTC 206 (S602). The input/output controller 210 then refers the setting information stored in the set value memory 218 to determine whether a graph of the present date and time is to be displayed (S603).

When the graph of the present date and time is to be displayed (YES in S603), the input/output controller210 executes a graph display process to display the graph on the display unit 209 (S604). The input/output controller 210 then waits for any command given through buttons or a time-out, for example, for five seconds (S605).

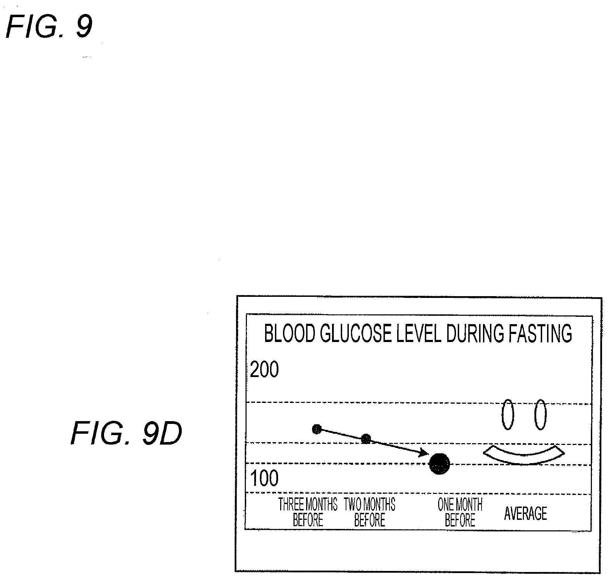

After executing the graph display process in step S604, the input/output controller 210 waits for any command given through buttons for five seconds at most in step S605. When five seconds have passed after step S604 or when a command is given through any button, the input/output controller executes a five-point rating display process to display a face illustration on the display unit 209 representing the five-point rating result (S606). The face illustrations in Figs. 9 will be described later. The input/output controller 210 then waits for any command given through buttons or a time-out, for example, for five seconds (S607).

After executing the five-point rating display process in step S606, the input/output controller 210 waits for any command given through buttons for five seconds at most in step S607. When five seconds have passed after step S606 or when a command is given through any button, the input/output controller finishes the series of process (S608).

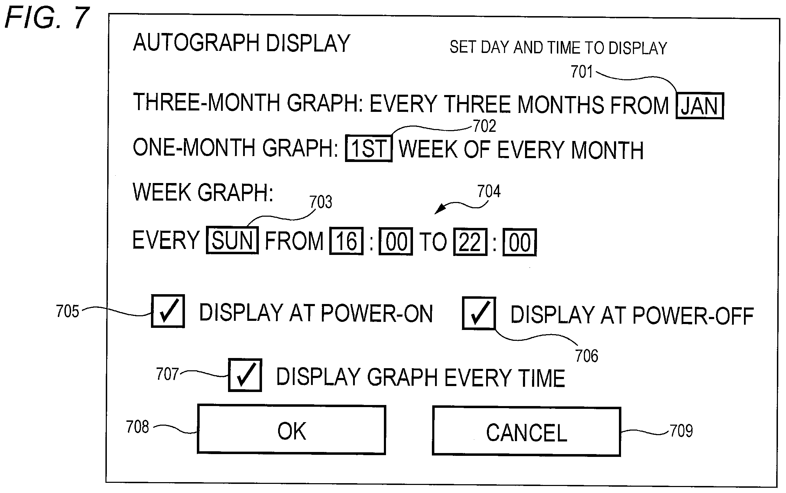

Fig. 7 illustrates an example of a setting screen for graph display on the liquid crystal display 103.

By execution of a setting process by the input/output controller 210 in step S512 in Fig. 5, setting screens are displayed on the display unit 209. One of the setting screens is a setting screen for graph display illustrated in Fig. 7.

In Fig. 7, a three-month graph input 701 is set to "1" to display a three-month graph every three months from January. When the three-month graph input 701 is set to "0", the three-month graph will not be displayed.

Similarly, a one-month graph input 702 is set to "1" to display a one-month graph in every first week of a month. When the one-month graph input 402 is set to "0", the one-month graph will not be displayed.

A day input 703 is set to "SUN" and a time zone input 704 is set to "16:00 - 22:00" to display a week graph every Sunday during 16:00 to 22:00. When the day input 703 is set blank, the week graph will not be displayed.

Further, a power-on check box 705, a power-off check box 706, and an every time check box 707 are checked to display the graphs at both timings of power-on and power-off every Sunday during 16:00 to 22:00.

The predetermined values set for inputs and check boxes described above are stored in the set value memory 218 when an OK button 708 is selected by pushing the determination button 107. The set values are canceled when a cancel button 709 is selected by pushing the determination button 107.

When the inputs and check boxes are set as mentioned above, the three-month graph, the one-month graph, and the week graph are all displayed at every timing of power-on and power-off on Sunday in the first week of January during 16:00 to 22:00.

From the second week to the final week of January, the week graph solely is displayed at every timing of power-on and power-off on Sunday during 16:00 to 22:00.

On Sunday during 16:00 to 22:00 in the first week of February, the one-month graph and the week graph are displayed at every timing of power-on and power-off.

On Sunday during 16:00 to 22:00 in the first week of April, the three-month graph, the one-month graph, and the week graph are all displayed at every timing of power-on and power-off.

The blood glucose meter 101, in an initial state, automatically recognizes the date of the first measurement (the day when a blood glucose level is first displayed) as a default setting to be stored in a ROM, according to which default setting the graphs are displayed. When the recognized date of the first measurement is April 16, 2012, the default setting for the graph display includes "4" in the three-month graph input 701, "3" in the one-month graph input 702, and "Mon" in the day input 703, which are automatically input. The default value of the time zone input 704 is "16:00 - 22:00".

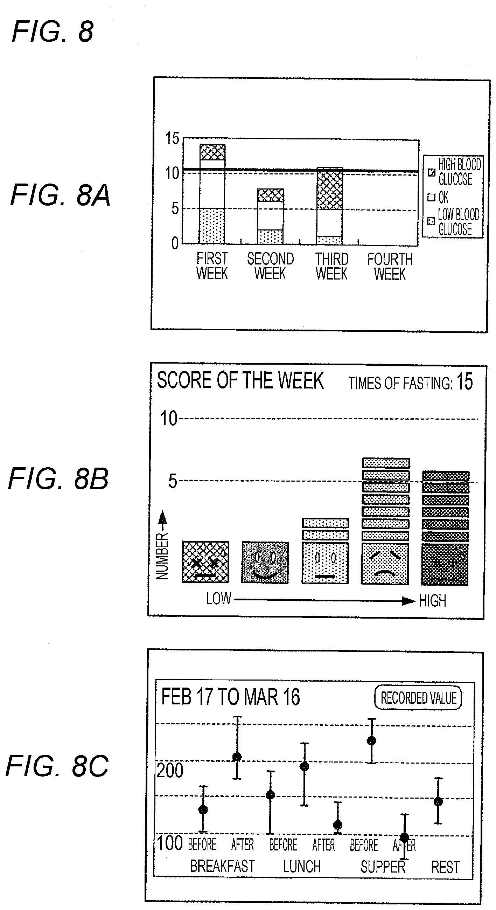

Figs. 8A to 8C and 9D are examples of the graph display on the liquid crystal display 103.

Fig. 8A illustrates the week graph. Weekly measured blood glucose levels are roughly categorized into three groups according to two thresholds. The number of measured "high blood glucose" (blood glucose level higher than an appropriate range), the number of measured "OK" (blood glucose level within the appropriate range), and the number of measured "low blood glucose" (blood glucose level lower than the appropriate range) are expressed in bar graphs.

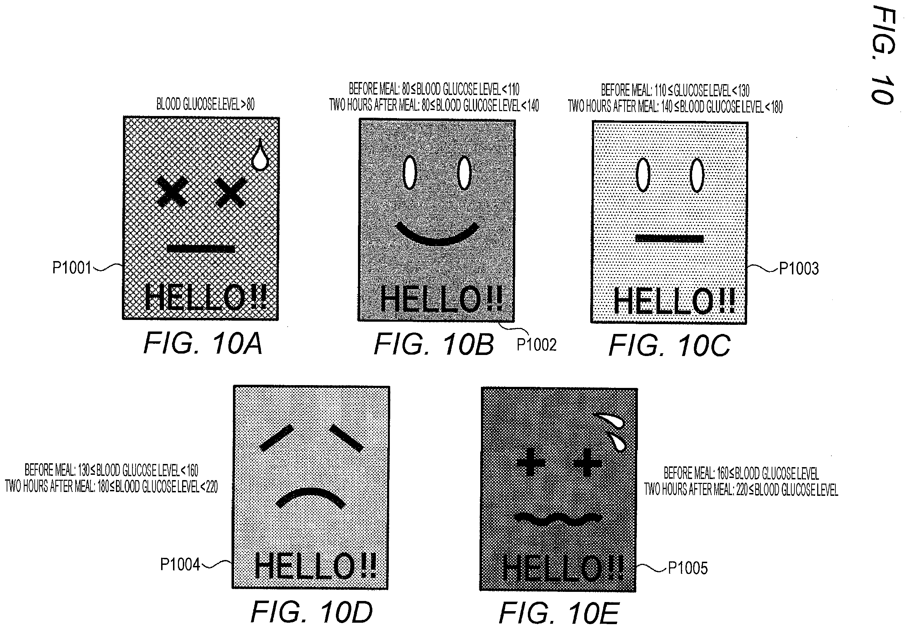

Fig. 8B illustrates a second display form of the week graph. Weekly measured blood glucose levels are compared with thresholds of five-point rating, which will be described later in Fig. 9, and summed results of the evaluation are expressed in bar graphs. The colors of bar graphs and face illustrations displayed with the bar graphs use the same designs and colors as the face illustrations in Figs. 10A to 10E.

Fig. 8C illustrates the one-month graph. Monthly measured blood glucose levels are categorized into groups of before-breakfast, after breakfast, before lunch, after lunch, before supper, after supper, and before rest. The average value, the maximum value, and the minimum value are calculated for each group. These values are then displayed in a form of a stock price chart.

Fig. 9D illustrates the three-month graph. Once in every three months, the average blood glucose level measured during fasting in the latest one-month period until the present, the average blood glucose level measured during fasting in a month period immediately preceding the latest one-month period, and the average blood glucose level measured during fasting in a month period immediately preceding the latest two-month period until the present are displayed in line graphs.

Figs. 10A to 10E are examples of face illustrations displayed by the five-point rating on the liquid crystal display 103.

The input/output controller 210 reads blood glucose levels measured during one to four week period until the present from the blood glucose level table 207, calculates the moving average value to compare with the five-point rating thresholds stored in the set value memory 218, and displays five-point rating results in the face illustrations.

The five-point rating is displayed in the face illustrations according to the thresholds and is in accordance with the "Guideline for Diagnosis of Diabetes" edited by The Japan Diabetes Society (http://www.lifescience.jp/ebm/cms/ms/no.19/topics.pdf).

Fig. 10A illustrates a face illustration indicating low blood glucose level. This is displayed when the blood glucose level measured during fasting and the blood glucose level measured two hours after a meal is lower than 80 mg/dl (deciliter). The face illustration P1001 is actually displayed in blue.

Fig. 10B illustrates a face illustration indicating an appropriate blood glucose level. This is displayed when the blood glucose level measured during fasting is within 80 to 110 mg/dl, and the blood glucose level measured two hours after a meal is within 80 to 140 mg/dl. The face illustration P1002 is actually displayed in yellow green.

Fig. 10C illustrates a face illustration indicating a slightly high blood glucose level. This is displayed when the blood glucose level measured during fasting is 110 mg/dl or higher and lower than 130 mg/dl, and the blood glucose level measured two hours after a meal is 140 mg/dl or higher and lower than 180 mg/dl. The face illustration P1003 is actually displayed in yellow.

Fig. 10D illustrates a face illustration indicating high blood glucose level. This is displayed when the blood glucose level measured during fasting is 130 mg/dl or higher and lower than 160 mg/dl, and the blood glucose level measured two hours after a meal is 180 mg/dl or higher and lower than 220 mg/dl. The face illustration P1004 is actually displayed in pink.

Fig. 10E illustrates a face illustration indicating dangerously high blood glucose level. This is displayed when the blood glucose level measured during fasting is 160 mg/dl or higher, and the blood glucose level measured two hours after a meal is 220 mg/dl or higher. The face illustration P1005 is actually displayed in red.

The values of the five-point rating thresholds are set differently for a timing before a meal and a timing two hours after a meal. Therefore, the blood glucose level measured before a meal and the blood glucose level after a meal cannot simply be added to calculate the average value. Thus, the blood glucose level measured two hours after a meal is converted into pseudo blood glucose level measured before meal.

The blood glucose level x can be converted into the blood glucose level measured before a meal using an additional value y by an equation expressed below, where a and b are a low limit and a high limit of blood glucose level measured two hours after a meal, respectively, and c and d are a low limit and a high limit of blood glucose level measured before a meal, respectively, and x is a blood glucose level measured two hours before a meal.

[Mathematical Formula 1]

$$y = \frac{(x-a)(d-c)}{b-a} + c$$

An example shown in Fig. 10C will be described below.

When the blood glucose level measured two hours after a meal is 155 mg/dl, a pseudo blood glucose level y measured before a meal is calculated by converting the blood glucose level x measured two hours after a meal by Mathematical Formula 1, where a = 140, b = 180, c = 110, d = 130, and x = 130.

$$(155 - 140) \times (130 - 110)/(180 - 140) + 130 = 137.5$$

mg/dl

That is, the blood glucose level measured two hours before a meal of 155 mg/dl can be converted into a pseudo blood glucose level measured before a meal of 137.5 mg/dl.

Further, the blood glucose level measured before a meal can be converted into a pseudo blood glucose level measured two hours after a meal to calculate an average value.

Furthermore, whether to include a value two hours after a meal in averaging can be selected by setting. That is, an average value before a meal, an average value after a meal, an average value before a meal and after a meal, or the like can be selected as required to be displayed.

The blood glucose meter 101 according to the embodiment described above can further be used in the following exemplary application.

(1) Five-point rating thresholds, namely, three before-meal thresholds and three two-hours-after-meal thresholds are stored in the set value memory 218. The number of thresholds can be smaller. However, at least two thresholds are necessary to show whether a blood glucose level is within an appropriate range. When two thresholds are given, the blood glucose meter 101 provides blood glucose level evaluation by three-point rating.

(2) The blood glucose meter 101 is configured to have the after-meal flag field in the blood glucose level table 207 to distinguish blood glucose levels between those before a meal and after a meal. The after-meal flag field is provided in the blood glucose level table 207 when the blood glucose meter 101 is in the "after-meal button" mode.

Instead of providing the after-meal flag field to the blood glucose level table 207, the blood glucose meter 101 is provided with a "meal button" mode to only record in the blood glucose level table 207 the information on date and time when a meal started, by a patient giving a long-push to the determination button 107 immediately before having a meal. The "after-meal button" mode and the "meal button" mode can be switched by changing the setting.

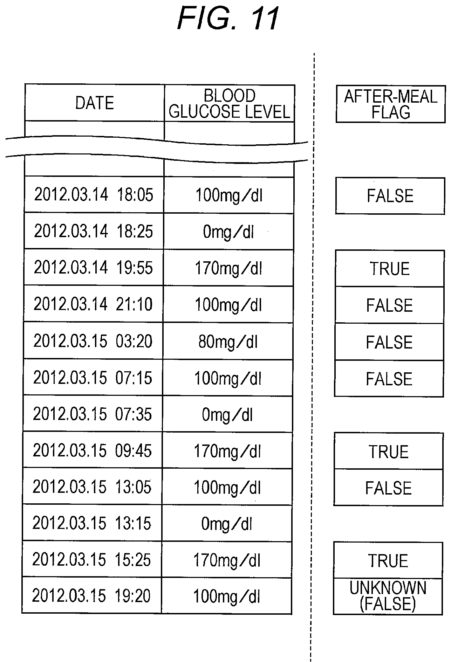

Fig. 11 is a schematic chart showing an example of a field structure and a record of the blood glucose level table 207 in the meal-button mode.

In the meal button mode, the after-meal flag field does not exist in the blood glucose level table 207. The blood glucose level value of "0", which is an unrealistic value (abnormal value), is instead recorded in the blood glucose level field. This meal record is recorded in the blood glucose level table 207 by pushing the meal button (giving a long-push to the determination button 107). The input/output controller 310 determines a record, which is taken immediately or within three hours after the meal record, as the after-meal record. The input/output controller 210 determines all the records of usual blood glucose level not conforming to this condition as before-meal records. Note that, the column denoted as "after-meal flag" in Fig. 10 illustrates how stored logical values are expressed when the after-meal flag field is active. Note that, non-numerical information may be recorded to express abnormal values.

Even when the blood glucose level table 207 is configured in this manner, the graph display and the five-point rating can be executed in the same manner.

(3) In a meal button mode, it is basically recommended to measure a blood glucose level and then push the meal button when having a meal. Some patients however have a lifestyle preferring a routine to push the meal button before measuring a blood glucose level and then take a meal. Thus, it is preferable to set timings to determine the blood glucose level measured before a meal and the blood glucose level after a meal based on the timing when the meal record is taken.

For example, determination can be made based on the timing one hour after taking the meal record. In such case, based on the timing one hour after taking the meal record, the blood glucose level measured in an hour-period immediately before taking the meal record is regarded as a value before a meal, and the blood glucose level measured in a three-hour-period immediately after taking the meal record is regarded as a value after a meal. For example, when a meal record is recorded at 11:30, based on the timing at 12:30 which is one hour past 11:30, the blood glucose level measured from 11:30 to 12:30 is regarded as a value before a meal and the blood glucose level measured from 12:30 to 15:30 is regarded as a value after a meal.

Other than the aspect described above, the blood glucose meter also functions as a computer processing biological information. The blood glucose meter is thus preferably configured to allow changing operational conditions and thresholds through a setting screen similar to that in Fig. 7 for each patient. Operational conditions and thresholds to be changed for each patient are, for example, whether to display all the graphs illustrated in Figs. 8A to 8C and Fig. 9, calculation ranges of average values of the five-point rating illustrated in Figs. 10A to 10E, and time ranges to determine before-meal values and after-meal values in the meal record mode.

A time range to determine before-meal values may preferably be an hour time period before taking the meal record and an hour time period after taking the meal record. A time range in which a measured blood glucose level is regarded as an after-meal value starts at the later one of the timing when the meal record is taken and the timing when the time range to determine the before-meal value finishes, and ends three hours after taking the meal record.

(4) The display of blood glucose level illustrated in Fig. 7, Figs. 8A to 8C, Fig. 9, and Figs. 10A to 10E on the liquid crystal display 103 provided by executing flowcharts illustrated in Figs. 4 to 6 can be provided by an independent computer.

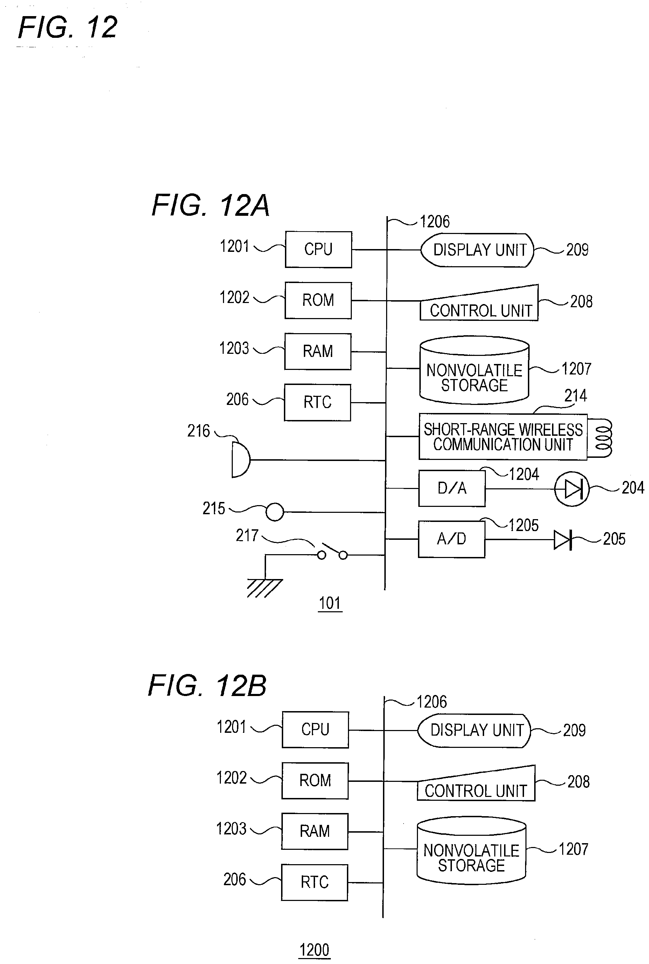

Figs. 12A and 12B illustrate block diagrams of the configuration of hardware including the blood glucose meter 101 and a personal computer 1200.

Fig. 12A is a block diagram illustrating a hardware configuration of the blood glucose meter 101.

In the blood glucose meter 101, which is an electronic device, including a well-known microcomputer as a major part, a bus 1206 connects a CPU 1201, a ROM 1202, a RAM 1203, the RTC 206, the control unit 208, the display unit 209, a nonvolatile storage 1207 containing the blood glucose level table 207, the set value memory 218, etc., the short-range wireless communication unit 214, the serial interface 215, the buzzer 216, the micro switch 217 assembled in the recess 112 to detect detachment of the cover 108, a D/A converter 1204 coupled to the LED 204, and an A/D converter 1205 coupled to the photodiode 205.

Fig. 12B illustrates a block diagram of the configuration of hardware of the personal computer 1200.

In the personal computer 1200, the bus 1206 connects the CPU 1201, the ROM 1202, the RAM 1203, the RTC 206, the control unit 208, the display unit 209, and the nonvolatile storage 1207. The hardware of the personal computer 1200 has an equivalent configuration as that of the blood glucose meter 101 not including the short-range wireless communication unit 214, the serial interface 215, the buzzer 216, the micro switch 217, the D/A converter 1204 coupled to the LED 204, and the A/D converter 1205 coupled to the photodiode 205.

Through the flowcharts illustrated in Figs. 4 to 6 executing related programs, the personal computer 1200 can also display the evaluation of blood glucose level illustrated in Fig. 7, Figs. 8A to 8C, Fig. 9, and Figs. 10A to 10E on the display unit 209. In other words, the function to measure blood glucose level is not necessary in the display function illustrated in Fig. 7, Figs. 8A to 8C, Fig. 9, and Figs. 10A to 10E. The display function requires data (blood glucose level table 207, etc.), a function of processing (input/output controller 210, etc.), the control unit 208, and the display unit 209.

That is, the personal computer 1200 may be configured to have hardware providing a function to display the evaluation of blood glucose level illustrated in Fig. 7, Figs. 8A to 8C, Fig. 9, and Figs. 10A to 10E on the display unit 209 through the flowcharts illustrated in Figs. 4 to 6 executing related programs.

The exemplary construction discloses the blood glucose meter 101.

When power is turned on and/or turned off, the blood glucose meter 101 reads the blood glucose level table 207 storing measured blood glucose levels and calculates the moving average value of the blood glucose level measured during a predetermined period of time until the present to evaluate the calculated result by the five-point rating. The evaluation result is displayed on the display unit 209 in any one of face illustrations P1001, P1002, P1003, P1004, and P1005 so that a patient can clearly understand the management status of his/her blood glucose level.

Further, the blood glucose meter 101 reads the blood glucose level table 207 storing measured blood glucose levels when power is turned on and/or turned off at a specific time in a specific day. Then an average value of the blood glucose level measured during a predetermined period of time until the present is calculated. By displaying a graph based on the average value on the display unit 209, a patient can clearly understand the management status of his/her blood glucose level.

The invention is defined in the appended claims. However, the present disclosure is not limited to the embodiment and may include other modifications and applications without departing from the scope of the present invention specified in the claims.

Reference Signs List

| 101 | blood glucose meter |
| 102 | housing |
| 103 | liquid crystal display |
| 104 | control button |
| 105 | up button |
| 106 | down button |
| 107 | determination button |
| 108 | cover |
| 109 | optical measurement unit |
| 110 | cap |
| 111 | set button |
| 120 | input/output controller |
| 201 | blood glucose level measuring unit |
| 202 | blood |
| 203 | test paper |
| 204 | LED |
| 205 | photodiode |
| 206 | RTC |

| 207 | blood glucose level table |
| 208 | control unit |
| 209 | display unit |
| 210 | input/output controller |
| 211 | large capacity storage |
| 212 | image file directory |
| 214 | short-range wireless communication unit |
| 215 | serial interface |
| 216 | buzzer |
| 217 | micro switch |
| 218 | set value |
| 220 | battery |
| 303 | test paper |
| 304 | LED |
| 306 | RTC |
| 307 | blood glucose level table |
| 308 | control unit |
| 309 | display unit |
| 310 | input/output controller |
| 313 | attribute table |
| 402 | one-month graph input |
| 701 | three-month graph input |
| 702 | one-month graph input |
| 703 | day input |
| 704 | time zone input |
| 705 | power-on check box |
| 706 | power-off check box |
| 707 | every time check box |
| 708 | OK button |
| 709 | cancel button |
| 1200 | personal computer |
| 1201 | CPU |
| 1202 | ROM |
| 1203 | RAM |
| 1204 | D/A converter |
| 1205 | A/D converter |
| 1206 | bus |
| 1207 | nonvolatile storage |

## Claims

1. A blood glucose meter (101) comprising:

a blood glucose level measuring unit (201) for measuring a blood glucose level of a patient;
a blood glucose level table (207, 307) including a blood glucose level field storing the blood glucose level of the patient measured with the blood glucose level measuring unit (201) and a date and time field storing a date and time when the blood glucose level measuring unit (201) measured the blood glucose level of the patient;
a display unit (209, 309) for displaying the blood glucose level stored in the blood glucose level field;
a control unit (208, 308) giving a command to supply power to the display unit (209, 309); and
an input/output controller (210, 310) configured to perform, in sequence, the power supply to the display unit (209, 309) according to a command given through the control unit (208, 308), reading the blood glucose level measured during a predetermined period of time from the blood glucose level table (207, 307) to calculate an average value, comparing the average value with a first threshold and a second threshold, and displaying image information on the display unit (209, 309) according to a compared result,

**characterized in that** the blood glucose level table (207, 307) includes an after-meal flag field indicating whether the patient measured a blood glucose level after a meal, and the input/output controller (210, 310) is configured to perform converting the blood glucose level related to an after-meal record where a logical value in the after-meal flag field is true into a pseudo blood glucose level measured before a meal, to calculate an average value.

2. The blood glucose meter (101) according to claim 1, wherein
the blood glucose level table (207, 307) indicates a moment immediately before a meal with an abnormal value set in the blood glucose level field as a meal record, and
the input/output controller (210, 310) converts the blood glucose level related to an after-meal record taken just after the meal record into the pseudo blood glucose levels to calculate the average value.

3. A program for operating a computer as an image information display apparatus comprising
a blood glucose level table (207, 307) including a blood glucose level field storing a blood glucose level of a patient measured with a blood glucose meter (101) and a date and time field storing a date and time when the blood glucose meter measured the blood glucose level of the patient,
a display unit (209, 309) for displaying a blood glucose level stored in the blood glucose level field,
a control unit (208, 308) giving a command to supply power to the display unit (209, 309), and
an input/output controller (210, 310) configured to perform, in sequence, the power supply to the display unit (209, 309) according to a command through the control unit (208, 308), reading the blood glucose level measured during a predetermined period of time from the blood glucose level table (207, 307) to calculate an average, comparing the average value with a first threshold and a second threshold, and displaying image information on the display unit (209, 309) according to a compared result,
**characterized in that** the blood glucose level table (207, 307) includes an after-meal flag field indicating whether the patient measured a blood glucose level after a meal, and the input/output controller (210, 310) is configured to perform converting the blood glucose level related to an after-meal record where a logical value in the after-meal flag field is true into a pseudo blood glucose level measured before a meal, to calculate an average value.

4. The program according to claim 3, wherein
the blood glucose level table (207, 307) indicates a moment immediately before a meal with an abnormal value set in the blood glucose level field as a meal record, and
the input/output controller (210, 310) converts the blood glucose level related to an after-meal record taken just after the meal record into the pseudo blood glucose levels to calculate the average value.


**Patentansprüche**

1. Blutzuckermessgerät (101), umfassend:

   eine Blutzuckerspiegel-Messeinheit (201) zum Messen eines Blutzuckerspiegels eines Patienten;
   eine Blutzuckerspiegel-Tabelle (207, 307), die ein Blutzuckerspiegel-Feld, das den mit der Blutzuckerspiegel-Messeinheit (201) gemessenen Blutzuckerspiegel des Patienten speichert, und ein Datums- und Zeitfeld umfasst, das ein Datum und eine Uhrzeit speichert, zu denen die Blutzuckerspiegel-Messeinheit (201) den Blutzuckerspiegel des Patienten gemessen hat;
   eine Anzeigeeinheit (209, 309) zum Anzeigen des im Blutzuckerspiegel-Feld gespeicherten Blutzuckerspiegels;
   eine Steuereinheit (208, 308), die einen Befehl zur Versorgung der Anzeigeeinheit (209, 309) mit Strom gibt; und
   eine Ein-/Ausgabesteuereinrichtung (210, 310), die so konfiguriert ist, dass sie hintereinander die Stromversorgung der Anzeigeeinheit (209, 309) gemäß einem durch die Steuereinheit (208, 308) gegebenen Befehl, das Auslesen des während einer vorbestimmten Zeitspanne gemessenen Blutzuckerspiegels aus der Blutzuckerspiegeltabelle (207, 307) zum Berechnen eines Durchschnittswertes, das Vergleichen des Durchschnittswertes mit einem ersten Schwellenwert und einem zweiten Schwellenwert und das Anzeigen der Bildinformationen auf der Anzeigeeinheit (209, 309) gemäß einem Vergleichsergebnis durchführt,
   **dadurch gekennzeichnet, dass** die Blutzuckerspiegel-Tabelle (207, 307) ein Nach-Mahlzeiten-Flagfeld enthält, das anzeigt, ob der Patient einen Blutzuckerspiegel nach einer Mahlzeit gemessen hat, und die Eingabe-/Ausgabesteuereinrichtung (210, 310) so konfiguriert ist, dass sie die Umwandlung des Blutzuckerspiegels, der mit einer Nach-Mahlzeiten-Aufzeichnung zusammenhängt, bei der ein logischer Wert in dem Nach-Mahlzeiten-Flagfeld wahr ist, in einen Pseudo-Blutzuckerspiegel, der vor einer Mahlzeit gemessen wurde, durchführt, um einen Durchschnittswert zu berechnen.

2. Blutzuckermessgerät (101) nach Anspruch 1, wobei
die Blutzuckerspiegel-Tabelle (207, 307) einen Moment unmittelbar vor einer Mahlzeit anzeigt, wobei ein abnormaler Wert in dem Feld für den Blutzuckerspiegel als Mahlzeiten-Aufzeichnung festgesetzt wird, und
die Eingabe-/Ausgabesteuereinrichtung (210, 310) den Blutzuckerspiegel, der mit einer unmittelbar nach der Mahlzeiten-Aufzeichnung aufgenommenen Nach-Mahlzeiten-Aufzeichnung zusammenhängt, in die Pseudo-Blutzuckerspiegel umwandelt, um den Durchschnittswert zu berechnen.

3. Programm zum Betreiben eines Computers als ein Bildinformationsanzeigegerät, umfassend:

eine Blutzuckerspiegel-Tabelle (207, 307), die ein Blutzuckerspiegel-Feld umfasst, das den mit einem Blutzuckermessgerät (101) gemessenen Blutzuckerspiegel eines Patienten speichert, und ein Datums- und Zeitfeld umfasst, das ein Datum und eine Uhrzeit speichert, zu denen das Blutzuckermessgerät den Blutzuckerspiegel des Patienten gemessen hat;
eine Anzeigeeinheit (209, 309) zum Anzeigen eines in dem Blutzuckerspiegel-Feld gespeicherten Blutzuckerspiegels;
eine Steuereinheit (208, 308), die einen Befehl zur Versorgung der Anzeigeeinheit (209, 309) mit Strom gibt; und
eine Ein-/Ausgabesteuereinrichtung (210, 310), die so konfiguriert ist, dass sie hintereinander die Stromversorgung der Anzeigeeinheit (209, 309) gemäß einem durch die Steuereinheit (208, 308) gegebenen Befehl, das Auslesen des während einer vorbestimmten Zeitspanne gemessenen Blutzuckerspiegels aus der Blutzuckerspiegeltabelle (207, 307) zum Berechnen eines Durchschnittswertes, das Vergleichen des Durchschnittswertes mit einem ersten Schwellenwert und einem zweiten Schwellenwert und das Anzeigen der Bildinformationen auf der Anzeigeeinheit (209, 309) gemäß einem Vergleichsergebnis durchführt,
**dadurch gekennzeichnet, dass** die Blutzuckerspiegel-Tabelle (207, 307) ein Nach-Mahlzeiten-Flagfeld enthält, das anzeigt, ob der Patient einen Blutzuckerspiegel nach einer Mahlzeit gemessen hat, und die Eingabe-/Ausgabesteuereinrichtung (210, 310) so konfiguriert ist, dass sie die Umwandlung des Blutzuckerspiegels, der mit einer Nach-Mahlzeiten-Aufzeichnung zusammenhängt, bei der ein logischer Wert in dem Nach-Mahlzeiten-Flagfeld wahr ist, in einen Pseudo-Blutzuckerspiegel, der vor einer Mahlzeit gemessen wurde, durchführt, um einen Durchschnittswert zu berechnen.

4. Programm nach Anspruch 3, wobei
die Blutzuckerspiegel-Tabelle (207, 307) einen Moment unmittelbar vor einer Mahlzeit anzeigt, wobei ein abnormaler Wert in dem Feld für den Blutzuckerspiegel als Mahlzeiten-Aufzeichnung festgesetzt wird, und
die Eingabe-/Ausgabesteuereinrichtung (210, 310) den Blutzuckerspiegel, der mit einer unmittelbar nach der Mahlzeiten-Aufzeichnung aufgenommenen Nach-Mahlzeiten-Aufzeichnung zusammenhängt, in die Pseudo-Blutzuckerspiegel umwandelt, um den Durchschnittswert zu berechnen.

**Revendications**

1. Indicateur de glycémie (101) comprenant :

une unité de mesure du niveau de glycémie (201) permettant de mesurer un niveau de glycémie d'un patient ;
un tableau de niveau de glycémie (207, 307) comprenant un champ de niveau de glycémie stockant le niveau de glycémie du patient mesuré à l'aide de l'unité de mesure du niveau de glycémie (201) et un champ de date et de temps stockant une date et une heure au moment où l'unité de mesure du niveau de glycémie (201) a mesuré le niveau de glycémie du patient ;
une unité d'affichage (209, 309) permettant d'afficher le niveau de glycémie stocké dans le champ de niveau de glycémie ;
une unité de commande (208, 308) donnant un ordre de fournir de l'énergie à l'unité d'affichage (209, 309) ; et
un dispositif de commande d'entrée/sortie (210, 310) conçu pour effectuer, en séquence, l'alimentation de l'unité d'affichage (209, 309) conformément à un ordre donné par l'unité de commande (208, 308), la lecture du niveau de glycémie mesuré pendant une durée prédéterminée à partir du tableau de niveau de glycémie (207, 307) afin de calculer une valeur moyenne, la comparaison de la valeur moyenne avec un premier seuil et avec un second seuil, et l'affichage d'informations d'image sur l'unité d'affichage (209, 309) conformément à un résultat comparé,
**caractérisé en ce que** le tableau de niveau de glycémie (207, 307) inclut un champ de drapeau après-repas indiquant si le patient a mesuré un niveau de glycémie après un repas, et le dispositif de commande d'entrée/sortie (210, 310) est conçu pour effectuer la conversion du niveau de glycémie par rapport à un enregistrement après-repas où une valeur logique dans le champ de drapeau après-repas est vraie en un pseudo-niveau de glycémie mesuré avant un repas, afin de calculer une valeur moyenne.

**2.** Indicateur de glycémie (101) selon la revendication 1, dans lequel
le tableau de niveau de glycémie (207, 307) indique un moment immédiatement avant un repas avec un ensemble de valeurs anormales dans le champ de niveau de glycémie en tant qu'enregistrement de repas, et
le dispositif de commande d'entrée/sortie (210, 310) convertit le niveau de glycémie par rapport à un enregistrement après-repas pris juste après l'enregistrement de repas en des pseudo-niveaux de glycémie afin de calculer la valeur moyenne.

**3.** Programme d'exploitation d'un ordinateur en tant qu'appareil d'affichage d'informations d'image comprenant :

un tableau de niveau de glycémie (207, 307) comprenant un champ de niveau de glycémie stockant un niveau de glycémie d'un patient mesuré à l'aide d'un indicateur de glycémie (101) et un champ de date et de temps stockant une date et une heure au moment où l'indicateur de glycémie a mesuré le niveau de glycémie du patient, une unité d'affichage (209, 309) permettant d'afficher un niveau de glycémie stocké dans le champ de niveau de glycémie,
une unité de commande (208, 308) donnant un ordre de fournir de l'énergie à l'unité d'affichage (209, 309), et un dispositif de commande d'entrée/sortie (210, 310) conçu pour effectuer, en séquence, l'alimentation de l'unité d'affichage (209, 309) conformément à un ordre donné par l'unité de commande (208, 308), la lecture du niveau de glycémie mesuré pendant une durée prédéterminée à partir du tableau de niveau de glycémie (207, 307) afin de calculer une valeur moyenne, la comparaison de la valeur moyenne avec un premier seuil et avec un second seuil, et l'affichage d'informations d'image sur l'unité d'affichage (209, 309) conformément à un résultat comparé,
**caractérisé en ce que** le tableau de niveau de glycémie (207, 307) inclut un champ de drapeau après-repas indiquant si le patient a mesuré un niveau de glycémie après un repas, et le dispositif de commande d'entrée/sortie (210, 310) est conçu pour effectuer la conversion du niveau de glycémie par rapport à un enregistrement après-repas où une valeur logique dans le champ de drapeau après-repas est vraie en un pseudo-niveau de glycémie mesuré avant un repas, afin de calculer une valeur moyenne.

**4.** Programme selon la revendication 3, dans lequel
le tableau de niveau de glycémie (207, 307) indique un moment immédiatement avant un repas avec un ensemble de valeurs anormales dans le champ de niveau de glycémie en tant qu'enregistrement de repas, et
le dispositif de commande d'entrée/sortie (210, 310) convertit le niveau de glycémie par rapport à un enregistrement après-repas pris juste après l'enregistrement de repas en des pseudo-niveaux de glycémie afin de calculer la valeur moyenne.

FIG. 1

FIG. 1A

FIG. 1B

FIG. 1C

2012.03.15 (THU) 11:50

PLEASE ATTACH CHIP.

FIG. 2

BLOOD GLUCOSE LEVEL MEASURING UNIT 201

202

204

203

205

BLOOD GLUCOSE LEVEL TABLE 207

SET VALUE MEMORY 218

220

INPUT/OUTPUT CONTROLLER 210

SHORT-RANGE WIRELESS COMMUNICATION UNIT 214

215

216

+Vcc

R219

217

RTC 206

CONTROL UNIT 208(104)

DISPLAY UNIT 209(103)

101

EP 2 843 413 B1

# FIG. 3

| DATE | BLOOD GLUCOSE LEVEL | AFTER-MEAL FLAG |
|---|---|---|
| | | |
| 2012.03.14 18:05 | 100mg/dl | FALSE |
| 2012.03.14 19:55 | 170mg/dl | TRUE |
| 2012.03.14 21:10 | 100mg/dl | FALSE |
| 2012.03.15 03:20 | 80mg/dl | FALSE |
| 2012.03.15 07:15 | 100mg/dl | FALSE |
| 2012.03.15 09:45 | 170mg/dl | TRUE |
| 2012.03.15 13:05 | 100mg/dl | FALSE |
| 2012.03.15 15:25 | 170mg/dl | TRUE |
| 2012.03.15 19:20 | 100mg/dl | FALSE |

# FIG. 4

```
                    ┌─────────────┐
                    │    START    │─── S401
                    └─────────────┘
                           │
                    ┌─────────────────┐
                    │ WAIT FOR COMMAND │─── S402
                    └─────────────────┘
                           │
                           │          S403
                      ╱─────────────╲        NO
                    ╱  POWER BUTTON   ╲──────────┐
                    ╲    PUSHED?      ╱          │
                      ╲─────────────╱            │
                           │ YES                 │
                           │          S404       │
                    ┌─────────────────┐          │
                    ║  MAIN PROCESS   ║─── S404   │
                    └─────────────────┘          │
                           │←────────────────────┘
                    ┌─────────────┐
                    │     END     │─── S405
                    └─────────────┘
```

FIG. 5

## FIG. 6

```
        ( START )──S601
           │
           ▼
   ┌─────────────────┐
   │ OBTAIN DATE AND │──S602
   │ TIME INFORMATION│
   └─────────────────┘
           │
           ▼
          ╱╲                    S603
         ╱  ╲
        ╱CONFORMING TO╲    NO
        ╲ CONDITION?  ╱─────────┐
         ╲          ╱           │
          ╲╱                    │
           │ YES                │
           ▼                    │
   ( DISPLAY GRAPH )──S604       │
           │                    │
           ▼                    │
   ┌─────────────────┐          │
   │ WAIT FOR COMMAND│──S605     │
   │   OR TIMEOUT    │          │
   └─────────────────┘          │
           │                    │
           │◄───────────────────┘
           ▼
   ( FIVE-POINT RATING DISPLAY )──S606
           │
           ▼
   ┌─────────────────┐
   │ WAIT FOR COMMAND│──S607
   │   OR TIMEOUT    │
   └─────────────────┘
           │
           ▼
        ( RETURN )──S608
```

## FIG. 7

AUTOGRAPH DISPLAY          SET DAY AND TIME TO DISPLAY

701

THREE-MONTH GRAPH: EVERY THREE MONTHS FROM [JAN]

702

ONE-MONTH GRAPH: [1ST] WEEK OF EVERY MONTH

WEEK GRAPH:          703          704

EVERY [SUN] FROM [16] : [00] TO [22] : [00]

705 — [✓] DISPLAY AT POWER-ON     [✓] DISPLAY AT POWER-OFF

706

707 — [✓] DISPLAY GRAPH EVERY TIME

708 — | OK |          | CANCEL | — 709

EP 2 843 413 B1

FIG. 8

FIG. 8A

FIG. 8B

FIG. 8C

FIG. 9

FIG. 9D

BLOOD GLUCOSE LEVEL DURING FASTING

200

100

THREE MONTHS
BEFORE
TWO MONTHS
BEFORE
ONE MONTH
BEFORE
AVERAGE

# FIG. 10

BLOOD GLUCOSE LEVEL > 80

**FIG. 10A**

P1001

BEFORE MEAL: 80 ≤ BLOOD GLUCOSE LEVEL < 110
TWO HOURS AFTER MEAL: 80 ≤ BLOOD GLUCOSE LEVEL < 140

**FIG. 10B** P1002

BEFORE MEAL: 110 ≤ GLUCOSE LEVEL < 130
TWO HOURS AFTER MEAL: 140 ≤ BLOOD GLUCOSE LEVEL < 180

P1003

**FIG. 10C**

BEFORE MEAL: 130 ≤ BLOOD GLUCOSE LEVEL < 160
TWO HOURS AFTER MEAL: 180 ≤ BLOOD GLUCOSE LEVEL < 220

P1004

**FIG. 10D**

BEFORE MEAL: 160 ≤ BLOOD GLUCOSE LEVEL
TWO HOURS AFTER MEAL: 220 ≤ BLOOD GLUCOSE LEVEL

P1005

**FIG. 10E**

# FIG. 11

| DATE | BLOOD GLUCOSE LEVEL | | AFTER-MEAL FLAG |
|---|---|---|---|
| 2012.03.14  18:05 | 100mg/dl | | FALSE |
| 2012.03.14  18:25 | 0mg/dl | | |
| 2012.03.14  19:55 | 170mg/dl | | TRUE |
| 2012.03.14  21:10 | 100mg/dl | | FALSE |
| 2012.03.15  03:20 | 80mg/dl | | FALSE |
| 2012.03.15  07:15 | 100mg/dl | | FALSE |
| 2012.03.15  07:35 | 0mg/dl | | |
| 2012.03.15  09:45 | 170mg/dl | | TRUE |
| 2012.03.15  13:05 | 100mg/dl | | FALSE |
| 2012.03.15  13:15 | 0mg/dl | | |
| 2012.03.15  15:25 | 170mg/dl | | TRUE |
| 2012.03.15  19:20 | 100mg/dl | | UNKNOWN (FALSE) |

# FIG. 12

## FIG. 12A

CPU 1201
ROM 1202
RAM 1203
RTC 206
216
215
217

1206
DISPLAY UNIT 209
CONTROL UNIT 208
NONVOLATILE STORAGE 1207
SHORT-RANGE WIRELESS COMMUNICATION UNIT 214
D/A 1204 — 204
A/D 1205 — 205

101

## FIG. 12B

CPU 1201
ROM 1202
RAM 1203
RTC 206

1206
DISPLAY UNIT 209
CONTROL UNIT 208
NONVOLATILE STORAGE 1207

1200

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009240127 A1
- EP 2345893 A1
- WO 2006072035 A1
- JP 2011064596 A